# EUROPEAN PATENT APPLICATION

(11) **EP 1 671 648 A1**
(43) Date of publication of application: **21.06.2006**
(21) Application number: 04788343.4
(22) Date of filing: 29.09.2004
(51) Int. Cl.: A61K 45/00, A61K 39/395, A61P 37/06, G01N 33/50, G01N 33/15

(54) **PREVENTION AND TREATMENT FOR GVHD**

(30) Priority: 02.10.2003 JP 2003345058
(71) Applicant: Eisai Co., Ltd., Tokyo 112-088 (JP)
(72) Inventor: MATSUSHIMA, Kouji, Matsudo-shi, Chiba 2710092 (JP); UEHA, Satoshi, Machida-shi, Tokyo 1940012 (JP); IMAI, Toshio, KAN Research Institute, Inc., Kyoto-shi, Kyoto 6008815 (JP)
(74) Representative: van Westenbrugge, Andries
(86) International application number: PCT/JP2004/014277
(87) International publication number: WO 2005/032589

(57) **Abstract**

The present invention is to provide a method for avoiding specifically and preventively or therapeutically an organ injury in graft-versus host disease (GVHD), a preventive or therapeutic agent therefore, and a method for screening the preventive or therapeutic agent. By inhibiting the interaction between fractalkine and the chemokine receptor CX3CR1 expressed on donor CD8 T cells, and by suppressing specifically organ infiltration by CD8 T cells to intestinal tract and the like, it is possible to prevent organ injuries in GVHD, and to treat and prevent GVHD selectively. A method using an anti-fractalkine antibody can be exemplified to inhibit the interaction between fractalkine and the chemokine receptor CX3CR1. Moreover, the present invention encompasses a method for screening a substance having a preventive or therapeutic effect on an organ injury in GVHD, by using a mechanism of the present invention for suppressing organ infiltration by CD8 T cells to intestinal tract or the like.

## Description

### Technical Field

The present invention relates to a method for avoiding preventively or therapeutically the onset of graft-versus-host disease (GVHD), particularly to a method for avoiding selectively, and preventively or therapeutically an organ injury in GVHD, a preventive or therapeutic agent specific to an organ injury in GVHD used therefor, and a method for screening a substance having a preventive or therapeutic effect specific to an organ injury in GVHD to be used as an active ingredient of the preventive or therapeutic agent.

### Background Art

Recent development of transplantation medicine is remarkable, and for example, bone marrow transplantations are drawing attention as a representative treatment method for various hematologic diseases including chronic myelogenous leukemia. In the field of transplantation medicine, intrinsically, there is a crucial problem wherein the immune system, which should be a defense reaction of the living body against harmful agents or organisms, reacts and induces rejection to transplantation or injury of host organs caused by the graft, as a defense reaction against the material transplanted to the host (recipient). A disease caused by an immune reaction reverse to the graft rejection, when immune cells of the graft react to host antigens, is called graft-versus-host disease (GVHD). When GVHD occurs, severe symptoms such as weight loss, diarrhea, vomiting, exfoliative dermatitis and hepatosplenomegaly are exhibited and may induce developmental arrest or even host death.

As above described, in the field of transplantation medicine, cell transplantation including bone marrow transplantation is drawing attention as a representative treating method to various hematologic diseases such as chronic myelogenous leukemia. However, GVHD which is a life-threatening complicated disease is also uncontrollable.

Human GVHD was first reported by Mathe et al. (Rev. Fr. Etud. Clin. Biol., 15:115-161, 1960). GVHD is fundamentally a clinical symptom of immunological reaction between donor cells and host tissues, and these clinical symptoms include eruption, gastrointestinal symptoms and hepatic disfunction, and are usually observed within two weeks from for example, allogeneic bone marrow transplantation. For the development of immune disorder, it is necessary that there is recognition of host antigen by immunologically competent donor cells, an immunocomprised host (recipient), and a difference of alloantigen between donor and recipient. It has been reported that immunologically competent donor cells are mature T cells, and that disease severity is associated with the number of T cells transplanted to host (NEJM 324:667, 1991).

Graft immunoreactions in organ transplantation can be classified into: acute rejections caused by a formation of sensitization after the first transplant; chronic rejections caused by a rupture of immune tolerance that has been once established; and hyperacute rejections caused by a promotion reaction by retransplantation and existing antibody just after retransplantation. It is believed that in acute GVHD, donor CD8 T cells react to histocompatibility antigen-mismatched host cells, proliferate, differentiate into cytotoxic T cells, and then infiltrate to skin, liver and intestinal tract which are the target organs to induce an organ injury (Nature 411: 385-389, 2001; Experimental Hematology 29: 259-277, 2001). It has been reported that among organ injuries in acute GVHD, intestinal GVHD is associated with the aggravation of GVHD, being the cause of systemic inflammation induced by bacterial translocation beyond the injured intestinal epithelia and the like with the dehydration from diarrhea (J. Clin. Invest. 104:317-325, 1999; J. Immunol. 152:1004-1011, 1994; Blood 94: 825-831, 1999; J. Clin. Invest. 107:1581-1589, 2001).

Current standard GVHD preventive protocols are non-specific immunosuppression by methotrexate or cyclosporine A (CsA), FK506 etc. targeting the proliferating process of immune cells ("Guideline for hematopoietic cell transplantation - Guideline of diagnosis and treatment of GVHD, Guideline committee for the Japan Society for Hematopoietic Cell Transplantation). Further, immunosuppressive agents such as corticosteroid, cyclophosphamide, and anti-thymocyte globulin (ATG) that effect widely are used for the prevention or treatment of GVHD. However, as these agents have a non-specific and a wide immunosuppressive effect, their toxicity is high and thus, infectious diseases due to compromised immune system or recurrence of tumor are becoming a problem. Therefore, development of an effective treating or preventing method for avoiding GVHD more selectively, and drugs therefor is currently awaited.

Recently, certain methods for preventing or treating GVHD or drugs therefor have been disclosed. For example, in Japanese Laid-Open Patent Application No. 9-188631, the use of a soluble Fas ligand suppressive agent comprising a compound with N-hydroxyaminocarbonyl group as a preventive or therapeutic agent of GVHD; in Published Japanese Translation of PCT International Publication No. 9-510607, the use of anti-B7-1 antibody or other B7-1 ligands for preventing or treating GVHD; and in Published Japanese Translation of PCT International Publication No. 11-508586, the use of a drug having GM-1 binding activity other than Ctx or Etx, or B-subunit of Ctx and Etx, or a drug that does not have GM-1 binding activity but affect GM-1 being mediated by intracellular signaling for preventing GVHD, are disclosed.

Further, in Published Japanese Translation of PCT International Publication No. 2002-505097, it is disclosed that molecule CD 147 which is expressed on particular cells such as T cells, B cells and monocytes, can be used for diseases such as GVHD; and in Published Japanese Translation of PCT International Publication No. 2003-512438, a method for inducing immune tolerance by using inhibitor of 11β-hydroxysteroid dehydrogenase in combination with mucosal administration of an antigen or administration of nucleic acid encoding the antigen is disclosed, as a method for inducing immune tolerance to control GVHD. However, these are still in a developmental stage, and neither effective methods for treating or preventing GVHD selectively nor drugs therefor has been found so far.

On the other hand, chemokine is known as a factor that controls various biological processes, for example, mobilization of immune cells to inflammatory sites, vasculogenesis sites, hematopoietic and organogenetic sites, in particular as a factor that is believed to be a regulator during trafficking process during immunity and inflammation for leukobyte. Chemokines are chemotactic cytokines released from wide variety of cells, and attract macrophages, T cells, eosinophils, basophils and neutropils to inflammatory sites (Cytokine, 3:165-183, 1991; Curr. Opin. Immunol., 6: 865-873, 1994; Rev. Immun., 12:593-633, 1994). Chemokines belong to a large family of proteins having single polypeptide chain of about 70 to 100 amino acids, and 50 and more members of the cytokine family is already known (FASEBJ., 3:2565-2573, 1989; Adv. Immunol., 55:97-179, 1994; Ann. Rev. Med., 50:425-440, 1999; New Eng. J. Med., 338:436-445, 1998).

Typically, known chemokines are classified into four subfamilies according to the location of the cysteine motif. Alpha chemokine CXC (α) is characterized by that the first two of the four cysteines are isolated by interstitial amino acids, and this subfamily mainly attracts neutrophils and lymphocytes. Beta-chemokine CC (β) is characterized by that no interstitial amino acid is present between the first two cysteines, and this subfamily mainly attracts macrophages, T cells, neutrophils and lymphocytes. Gamma-chemokine C (γ) is characterized by a single C residue, and shows specificity to lymphocytes. Delta-chemokine CX3C (δ) is characterized by a cysteine pair separated by 3 residues, and shows specificity to lymphocytes and monocytes. Chemokines bind to a specific cell surface receptor belonging to G protein-coupled 7 trans-membrane domain protein, and play a biological role by interaction with the chemokine receptor.

The chemokine receptor can bind to a certain subfamily of chemokine and the name of the receptor reflects the binding characteristic limited to chemokine subfamily. For example, receptors binding to CXC chemokine are indicated as CXCR, and receptors binding to CC chemokine are indicated as CCR. The number representing the order in which the particular receptor has been found is shown after the indication of the subfamily specific receptor. For example, CXCR4 represents to be the 4th to be found as CXCR.

Recently, fractalkine (FKN) has been identified and reported as a new chemokine in the chemokine super family (Nature, 385 (6617): 640-644, 1997). Fractalkine has a mucin-chemokine hybrid type structure with CX3C chemokine motif and mucin-like domain, and exist as two distinct types, that is, membrane-binding type and secretory type. Membrane-binding type fractalkine is strongly induced to be expressed on the cell surface of vascular endothelial cells by stimulation of inflammatory cytokines, and induces cell adhesion of NK cells and CD8-positive T cells. On the other hand, fractalkine that has become secretory type by being cleaved by a protease at a processing site proximal to transmembrane region of fractalkine protein induces migration of these leukocytes.

Similarly to other chemokine receptors, CX3CR1 is a 7 trans-membrane G protein coupled-receptor, and has been identified as a fractalkine receptor (Cell, 91(4):521-530, 1997). CX3CR1 is expressed on the cell surface of NK cells or CD8-positive T cells, and mediates both migration and adhesion of these leukocytes via fractalkine binding.

The object of the present invention is to provide a method for avoiding preventively or therapeutically an onset of graft-versus-host disease (GVHD), particularly a method for avoiding selectively, and preventively or therapeutically an organ injury in GVHD, a preventive or therapeutic agent specific for an organ injury in GVHD used therefor, and a method for screening a substance having a preventive or therapeutic effect specific to an organ injury in GVHD to be used as an active ingredient of the preventive or therapeutic agent.

The present inventors made a keen study to find out a method for avoiding preventively or therapeutically an onset of graft-versus-host disease (GVHD), for avoiding specifically an organ injury in GVHD, and found out that by inhibiting an interaction between fractalkine and the chemokine receptor CX3CR1 which is expressed on donor CD8 T cells, and by suppressing specifically organ infiltration by CD8 T cells to intestinal tract, etc., it is possible to prevent organ injuries in GVHD and to perform selective treatment and prevention of GVHD. The present invention has been thus completed.

In other words, the present inventors focused on that organ injuries in acute GVHD are limited to skin, kidney and intestinal tract, and considered the organ-specific infiltration by donor CD8 T cells that has acquired cell toxicity in the late phase of GVHD to be its mechanism, and thought that by suppressing the infiltration process, selective treatment and prevention of GVHD would be possible. Thus, in the present case, the present inventors used an acute GVHD model (J. Immunology, 155: 2396-2406, 1995; J. Immunology, 161: 2848-2855, 1998) and performed experiments to inhibit the interaction between fractalkine and the chemokine receptor CX3CR1 which is expressed on donor CD8 T cells by anti-fractalkine antibody administration. They found out that an interaction between fractaline which is expressed in intestinal tract epithelium and vascular endothelium and CX3CR1 which is expressed on donor CD8 T cells plays a critical role in their intestinal infiltration in the late phase of GVHD, and that by inhibiting the interaction, intestinal GVHD can be reduced.

In the present invention, inhibition of the interaction between fractalkine and the chemokine receptor CX3CR1 which is expressed on donor CD8 T cells for suppressing specifically organ infiltration by CD8 T cells, can be performed by inhibiting the function of fractalkine and/or the chemokine receptor CX3CR1 which is expressed on donor CD8 T cells. As for a method for inhibiting the function of fractalkine and/or the chemokine receptor CX3CR1, for example, a method for inhibiting the expression of fractalkine or the chemokine receptor CX3CR1 by genetic engineering or a method using an antagonist inhibitor of the chemokine receptor CX3CR1 can be exemplified. A method using an anti-fractalkine antibody for inhibiting the interaction between fractalkine and the chemokine receptor CX3CR1 which is expressed on donor CD T cells can be especially preferably exemplified.

The present invention further encompasses a method for screening substances having a preventive or therapeutic effect specific to an organ injury in graft-versus-host disease by administrating a test substance to an animal model with graft-versus host disease transferred with donor CD8 T cells, and to detect/evaluate the infiltration by donor CD8 T cells to organs. In the screening method, the infiltration by donor CD8 T cells to organs can be determined by detecting/evaluating the expression of the chemokine receptor CX3CR1 in donor CD8 T cells and/or fractalkine.

### Disclosure of the Invention

In other words, specifically, the present invention relates to a method for avoiding preventively or therapeutically an onset or a development of graft-versus-host disease by suppressing specifically an organ infiltration by CD8 T cells ("1"); the method for avoiding preventively or therapeutically an onset or a development of graft-versus-host disease according to "1", wherein the specific suppression of an organ infiltration by CD8 T cells is inhibition of the interaction between fractalkine and a chemokine receptor CX3CR1 which is expressed on donor CD8 T cells ("2"); the method for avoiding preventively or therapeutically an onset or a development of graft-versus-host disease according to "2", wherein the inhibition of the interaction between fractalkine and the chemokine receptor CX3CR1 is inhibition of a function of fractalkine and/or a chemokine receptor CX3CR1 which is expressed on donor CD8 T cells ("3"); the method for avoiding preventively or therapeutically an onset or a development of graft-versus-host disease according to "2" or "3", wherein the inhibition of the interaction between fractaline and the chemokine receptor CX3CR1 which is expressed on donor CD8 T cells is the inhibition of the interaction using an anti-fractalkine antibody ("4"); the method for avoiding preventively or therapeutically an onset or a development of graft-versus-host disease according to "4", wherein the anti-fractalkine antibody is a monoclonal antibody ("5"); the method for avoiding preventively or therapeutically an onset or a development of graft-versus-host disease according to any one of "1" to "5", wherein the specific suppression of an organ infiltration by CD8 T cells is an infiltration by cytotoxic CD8 T cells to intestinal tract ("6").

Moreover, the present invention relates to a preventive or therapeutic agent specific to an organ injury in graft-versus-host disease, comprising a substance inhibiting an interaction between fractalkine and the chemokine receptor CX3CR1 which is expressed on donor CD8 T cells as an active component ("7"); the preventive or therapeutic agent specific to an organ injury in graft-versus-host disease according to "7", wherein the substance inhibiting an interaction between fractalkine and the chemokine receptor CX3CR1 which is expressed on donor CD8 T cells is a substance inhibiting a function of fractalkine and/or the chemokine receptor CX3CR1 which is expressed on donor CD8 T cells ("8"); the preventive or therapeutic agent specific to an organ injury in graft-versus-host disease according to "7" or "8", wherein the substance inhibiting the interaction between fractaline and the chemokine receptor CX3CR1 which is expressed on donor CD8 T cells is an anti-fractalkine antibody ("9"); the preventive or therapeutic agent specific to an organ injury in graft-versus-host disease according to "9", wherein the anti-fractalkine antibody is a monoclonal antibody ("10").

Furthermore, the present invention relates to a method for screening a substance having a preventive or therapeutic effect specific to an organ injury in graft-versus-host disease, comprising the steps of administering a test substance to a model animal with graft-versus-host disease transferred with donor CD8 T cells, and detecting/evaluating an infiltration by donor CD8 T cells to organs ("11") ; the method for screening a substance having a preventive or therapeutic effect specific to an organ injury in graft-versus-host disease according to "11", wherein the infiltration by donor CD8 T cells to organs is performed by detecting/evaluating the expression of the chemokine receptor CX3CR1 and/or fractalkine in donor CD8 T cells ("12"); the method for screening a substance having a preventive or therapeutic effect specific to an organ injury in graft-versus-host disease according to "11" or "12", wherein the infiltration by donor CD8 T cells to organs is an infiltration by donor CD 8 T cells to intestinal tract ("13"); the method for screening a substance having a preventive or therapeutic effect specific to an organ injury in graft-versus-host disease according to any one of "11" to "13", wherein the model animal with graft-versus-host disease transferred with donor CD8 T cells is a model mouse with graft-versus-host disease transferred with donor CD8 T cells ("14"); a preventive or therapeutic agent specific to an organ injury in graft-versus host disease comprising a substance obtained by the method for screening substances having a preventive or therapeutic effect specific to an organ injury in graft-versus-host disease according to any one of "11" to "14" as an active component ("15").

### Brief Description of Drawings

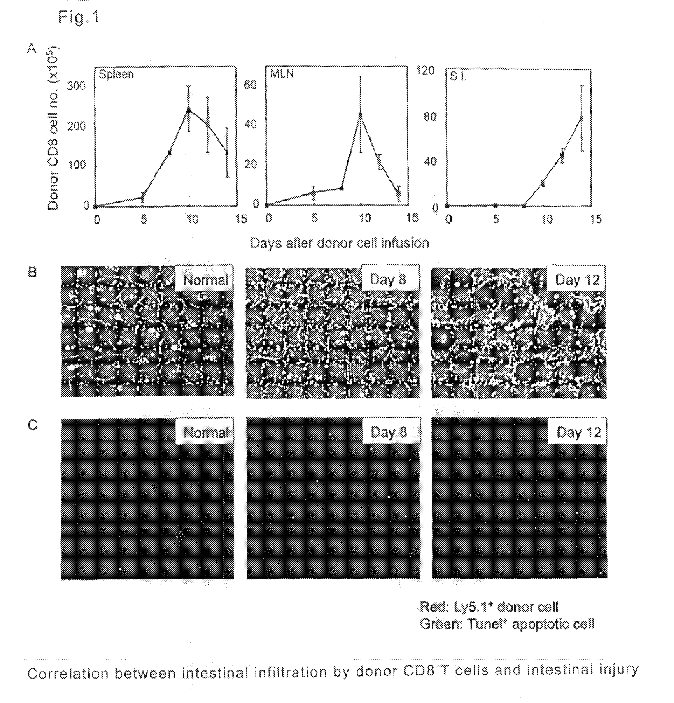

Fig. 1 is a figure showing the results of investigating the correlation between intestinal infiltration by donor CD8 T cells and intestinal injury, in the examples of the present invention. A shows the infiltration by donor CD8 T cells in spleen, mesenteric lymph node, small intestine; B shows the image of intestinal tissue (HE staining) of normal BDF1 and on day 8 and day 12 after induction of GVHD; C shows the detection of apoptotic cells in intestinal tract of normal BDF1 and on day 8, day 12 after induction of GVHD.

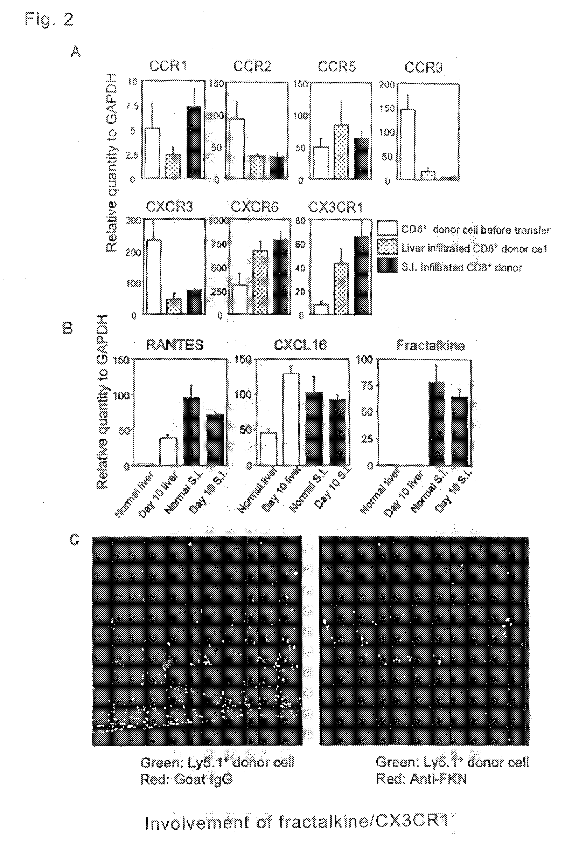

Fig. 2 is a figure showing the results of investigating the involvement of fractalkine/CX3CR1 in intestinal GVHD, in the examples of the present invention. A shows gene expression of the chemokine receptor in donor CD8 T cells before transfer and those infiltrated in intestinal tract and liver on day 10 after GVHD induction. B shows gene expression of the chemokine in intestinal tract and liver of normal and on day 10 after GVHD induction. C shows fractalkine expression and infiltration of donor lymphocyte in intestinal tract on day 10 after GVHD induction.

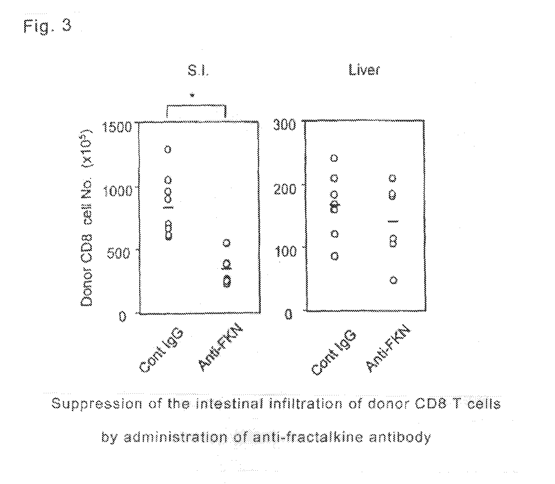

Fig. 3 is a figure showing the results of investigating the suppression of intestinal infiltration of donor CD8 T cells by administrating an anti-fractalkine antibody, in the examples of the present invention.

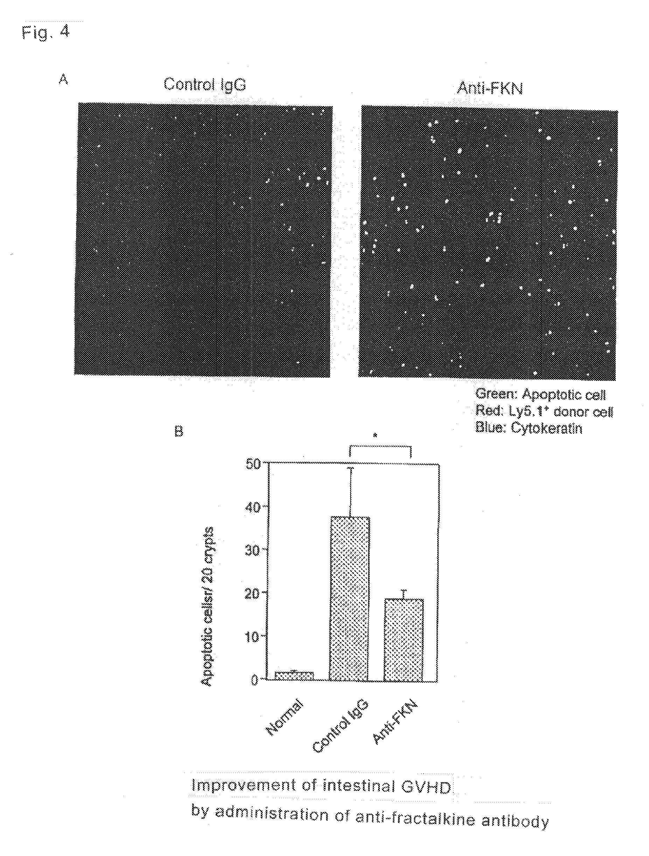

Fig. 4 is a figure showing the results of investigating the improvement in intestinal GVHD by administrating an anti-fractalkine antibody, in the examples of the present invention. A shows the detection of apoptotic cells in intestinal crypt by Tunel staining, B shows the number of apoptotic cells per 20 intestinal crypts.

### Best Mode of Carrying Out the Invention

The present invention relates to a method for avoiding preventively or therapeutically an onset or development of GVHD, by inhibiting an interaction between fractalkine, a chemokine, and the chemokine receptor CX3CR1 which is expressed on donor CD8 T cells, or the like, to suppress specifically organ infiltration by CD8 T cells.

Fractalkine and the chemokine receptor CX3CR1 are known proteins (Nature, 385 (6617): 640-644, 1997; Cell, 91(4):521-530, 1997), and sequences of the genes and the proteins can be accessed with the accession numbers NM_001337 and NM_002996 in GenBank database.

As for a method for inhibiting the interaction between fractalkine and the chemokine receptor CX3CR1 which is expressed on donor CD8 T cells can be performed by inhibiting each of the function of fractalkine and the chemokine receptor CX3CR1, and known methods can be used for inhibiting these functions. With the method, it is possible to control the gene expression of fractalkine or of the chemokine receptor CX3CR1 which is expressed on donor CD8 T cells by genetic engineering, and to inhibit each of the function of fractalkine and the chemokine receptor CX3CR1. Methods for controlling the expression by known genetic engineering such as antisense method can be used for controlling the gene expression. In other words, gene expression can be controlled by constructing an antisense strand of the sequence according to known sequences of fractalkine and the chemokine receptor CX3CR1, and by introducing the sequence into the cells.

Further, by using an antagonist of the chemokine receptor CX3CR1, the binding to CX3CR1 receptor of fractalkine which is its ligand can be antagonistically inhibited and the interaction between fractalkine and the chemokine receptor CX3CR1 can be thus inhibited. As for a particularly preferable method for inhibiting the interaction between fractalkine and the chemokine receptor CX3CR1, a method using an anti-fractalkine antibody can be exemplified. As for the antibodies, monoclonal and polyclonal antibodies can be used. These antibodies can be prepared by sensitizing mammals (mice, hamsters, rabbits, etc.) with the use of fractalkine proteins by a known method (Japanese Laid-Open Patent Application No. 2002-345454).

Further, the present invention encompasses a method for screening a substance having a preventive or therapeutic effect specific to an organ injury, by using a mechanism that develops an organ injury in GVHD, which was found in the present invention. The method for screening of the present invention can be performed by administrating a test substance to a model animal with GVHD transferred with donor CD8 T cells, and by detecting/evaluating infiltration by donor CD8 T cells to organs to evaluate the effect of the test substance. Administration of a test substance to a model animal with GVHD transferred with donor CD8 T cells, and detection/measurement of infiltration by donor CD8 T cells to organs, can be performed by administrating a test substance to the model animal, incising the tissues, and by directly detecting/measuring of histologically the infiltration by donor CD8 T cells to tissues, by using immunofluorescent histological staining with the use of antibodies, etc.

Moreover, infiltration by donor CD8 T cells to tissues can be determined by detecting/evaluating the expression of the chemokine receptor CX3CR1 and/or fractalkine in donor CD8 T cells. Detection/evaluation of the expression of the chemokine receptor CX3CR1 and/or fractalkine in donor CD8 T cells can be performed by a method for detecting gene expression by known genetic engineering. For example, the expression of the chemokine receptor CX3CR1 gene and fractalkine gene can be dectected by constructing a probe from known gene sequences of the chemokine receptor CX3CR1 and fractalkine, to detect the expression of the chemokine receptor CX3CR1 gene and fractalkine gene by using the probe. Further, detection/evaluation of the expression of the chemokine receptor CX3CR1 and/or fractalkine can be performed by an antigen-antibody method using antibodies against the protein. Fluorescent antibody method can be exemplified for a preferable example of the antigen-antibody method.

Administration of the preventive or therapeutic agent specific to an organ injury in GVHD of the present invention to the living body can be performed by formulating an active component of the preventive or therapeutic agent of the present invention, with auxiliary components comprising known pharmaceutically acceptable carriers or diluents and by administrating them orally or parenterally. For parenteral administration, as intravenous, intramuscular and subcutaneous administrations are performed, for example, the formulation is formed as an injectable form. Further, it can be formulated in a state that a mucosal administration is possible, so that administration through mucosa is possible.

In the following, the present invention will be explained in detail with reference to the examples, while the technical scope of the present invention is not limited to these examples.

### Example 1

### [Material and methods]

### (Mice)

C57BL/6 mice (B6:H2b) were purchased from Clea Japan.

(C57BL/6 × DBA/2) F1 mice (BDF1:Ly5.2, H2b × d) were purchased from Clea Japan. GFP transgenic B6 mice (GFP-B6) were provided by Dr. Okabe (Osaka University). Ly5. 1 congenic B6 mice (Ly.5.1-B6) were provided by Dr. Ishikawa (Keio University). All of the animal experiments were performed according to the guideline of animal experiments of University of Tokyo. Female mice between the ages of 8 to 12 weeks were used for the experiments.

### (Antibodies)

Biotin-labeled mouse anti-Ly5.1 monoclonal antibody, PE-conjugated rat anti-mouse CD8 monoclonal antibody, purified rat anti-mouse CD 16/32 monoclonal antibody were purchased from BD PharMingen; purified goat-polyclonal anti-mouse fractalkine antibody was purchased from R&D; purified rabbit polyclonal anti-cytokeratin antibody was purchased from Biomedical Technologies Inc.; Alexa 546-labeled anti-goat IgG antibody, Alexa 488-labeled Streptavidin were purchased from Molecular probe. Purified hamster monoclonal anti-fractalkine antibody (5H8-4, Japanese Laid-Open Patent Application No: 2002-345454) was provided by Dr. Imai (Kan Research Institute Inc.).

### (Preparation of lymphocytes)

Splenocytes: Spleens were prepared from mice sacrificed by cervical dislocation, ground in PBS to prepare cell suspension. After washing by centrifugation, pellets were re-suspended in ACK buffer (0.15 M NH4Cl, 1 mM KHC03, 0. 1 mM Na2EDTA pH 7.4, 1 ml/mouse), to remove red blood cells by hemolysis. After washing by centrifugation with PBS, cell aggregates were removed by passing through nylon mesh (pore size 70 µm) to prepare splenocytes.

Lymphocytes of the mesenteric membrane lymph nodes: After collecting mesenteric lymph nodes, fat tissues were removed, ground in PBS to prepare cell suspension. After centrifugation, cell aggregates were removed by passing through nylon mesh (pore size 70 µm), to prepare lymphocytes of the mesenteric membrane lymph node.

Intestinal lymphocytes: Intestinal tract was collected from pylorus to ileal terminal, and then reversed after removing the mesentery. Intestinal tract was divided into four pieces avoiding the Peyer's patch, and transferred into a 50 ml-conical tube filled with 45 ml RPMI-1640/5% FBS/20 mM HEPES. The sample was shaken horizontally at 37°C, 160 rpm, for 45 min. Cell aggregates were removed from the obtained supernatant by passing through a nylon wool column, to prepare single cell suspension. After centrifugation, cells were re-suspended into 45% Percoll (Amersham Bioscience), and after putting 75% Percoll in lower layer, the sample was centrifuged at RT, 2000 rpm for 20 min. Lymphocyte fraction was collected from intermediate layer of 45%/75% Percoll, to prepare intestinal lymphocytes.

Liver lymphocytes: Liver was prepared from mice sacrificed by bleeding, ground in RPMI-1640/5% FBS, to prepare cell suspension by passing through stainless mesh (pore size 70 µm). After washing by centrifugation, cell pellet was re-suspended in 33% Percoll, centrifuged at RT, 2000 rpm, for 10 min. After removing supernatant containing hepatocytes, cell pellet was re-suspended in 1 ml-ACK buffer, and red blood cells were lysed. After centrifugation, the obtained cell pellet was re-suspended in PBS to prepare liver lymphocytes.

### (Acute GVHD model)

Spleen cells (6 × 10⁷) of GFP-B6 (for FCM analysis) or Ly.5.1-B6 (for tissue staining) were intravenously injected into BDF1 mice without radiation. In order to perform a time-course analysis of organ infiltration by donor lymphocytes, lymphocytes were prepared from spleen, mesenteric lymph nodes, small intestine obtained from the BDF1 mice on day 5, 8, 10, 12, 14 after transferring donor cells. After measuring the number of viable cells by Trypan Blue staining, Flow-cytometric (FCM) analysis was performed and the number of donor CD8 T cells was calculated from the ratio of GFP-positive or CD8-positive cells.

### (FCM analysis)

Anti-mouse CD16/32 antibody (0.01 µg/2 × 10⁵ cells) was added to cell suspension, incubated at 4°C, for 10 min, to inhibit non-specific binding via Fc receptor. Then, PE-labeled anti-mouse CD8 antibody (0.05 µg/2 × 10⁵ cells) was added, incubated at 4°C, for 20 min. Further, PBS was added and centrifuged. FCM analysis was performed by EPICS ELITE (Beckman coulter) and dead cells were removed by PI staining (final concentration 0.5 µg/ml).

### (Evaluation of intestinal GVHD)

Intestinal tissue was dissected in longitudinal direction, embedded in OCT compound (Tissue-teck) being trimmed to a 5 µm-square sheet-like and frozen in liquid nitrogen. Frozen sections with a thickness of 6 µm were prepared with Cryostat, and the tissue image of intestinal crypts was evaluated by HE staining. Further, *in situ* cell death detection kit (Roche) was used. With the combination of Tunel and immunofluorescent histological staining using anti-cytokeratin antibody and anti-Ly5.1 antibody, apoptosis of epidermis and infiltration by donor cells were detected in intestinal crypts. Further, in order to quantify intestinal injury, the number of apoptotic cells per 20 crypts was calculated. (RT-PCR)

Donor CD8 T cells before transfer, and those (1-10 × 10⁴ cells) infiltrated to intestinal tract or liver on day 10 after GVHD induction were separated with a cell sorter, and total RNA was prepared by using TRIzol reagent (Invitrogen). Further, liver and small intestine were collected from normal BDF1 mice and those on day 10 after GVHD induction, and total RNA was prepared by using TRIzol reagent (Invitrogen). The obtained RNA (200 ng) was reverse-transcribed to cDNA by using PowerScript Reverse Transcrpitase (Clontech) and random hexamers (Promega). Various primers and TaqMan probe, Platinum quantitative PCR PuperMix-UDG (Invitrogen) were admixed with a fraction of the cDNA as a template to prepare a reaction solution. Real time PCR was performed with ABI PRISM 7700 (Applied biosystems) according to the attached manual. Composition of the reaction solution and PCR conditions were set according to the manual attached to Platinum quantitative PCR PuperMix-UDG. Expressions of the chemokine receptor/chemokine gene are shown by a relative level, with GAPDH as an internal standard. Various primers and TaqMan probe used are shown in Table 1.

**(Table 1) Primers and TaqMan Probe sequences for Real-Time PCR**

| Gene | Sequence (5' - 3') | | | | | | | | | |
|---|---|---|---|---|---|---|---|---|---|---|
| mGAPDH sense | AGT | ATG | ACT | CCA | CTC | ACG | GCA | A | | |
| mGAPDH anti-sense | TCT | CGC | TCC | TGG | AAG | ATG | GT | | | |
| mGAPDH TaqMan probe | AAC | GGC | ACA | GTC | AAG | GCC | GAG | AAT | | |
| CXCR3 sense | ATC | AGC | GCT | TCA | ATG | CCA | C | | | |
| CXCR3 anti-sense | TGG | CTT | TCT | CGA | CCA | CAG | TT | | | |
| CXCR3 TaqMan probe | ATG | CCC | ATA | TCC | TAG | CTG | TTC | TGC | TGG | TC |
| CCR1 sense | GTG | TTC | ATC | ATT | GGA | GTG | GTG | G | | |
| CCR1 anti-sense | GGT | TGA | ACA | GGT | AGA | TGC | TGG | TC | | |
| CCR1 TaqMan probe | TGG | TGC | TCA | TGC | AGC | ATA | GGA | GGC | TT | |
| CCR2 sense | TGT | TAC | CTC | AGT | TCA | TCC | ACG | G | | |
| CCR2 anti-sense | CAG | AAT | GGT | AAT | GTG | AGC | AGG | AAG | | |
| CCR2 TaqMan probe | TCT | GCT | CAA | CTT | GGC | CAT | CTC | TGA | CC | |
| CCR5 sense | CAT | CGA | TTA | TGG | TAT | GTC | AGC | ACC | | |
| CCR5 anti-sense | CAG | AAT | GGT | AGT | GTG | AGC | AGG | AA | | |
| CCR5 TaqMan probe | TAC | CTG | CTC | AAC | CTG | GCC | ATC | TCT | GA | |
| CCR9 sense | CCA | TTC | TTG | TAG | TGC | AGG | CTG | TT | | |
| CCR9 anti-sense | AAG | CTT | CAA | GCT | ACC | CTC | TCT | CC | | |
| CCR9 TaqMan probe | ACG | CTT | ATG | CCA | TGT | TCA | TCT | CCA | ACT | GC |
| CX₃CR1 sense | CCG | CCA | ACT | CCA | TGA | ACA | A | | | |
| CX₃CR1 anti-sense | CGT | CTG | GAT | GAT | GCG | GAA | GTA | | | |
| CX₃CR1 TaqMan probe | CGT | CAC | CCC | AGT | TCA | TGT | TCA | CAA | ATA | G |
| RANTES sense | CAT | ATG | GCT | CGG | ACA | CCA | CT | | | |
| RANTES anti-sense | ACA | CAC | TTG | GCG | GTT | CCT | TC | | | |
| RANTES TaqMan probe | CAA | GTG | CTC | CAA | TCT | TGC | AGT | CGT | G | |
| mCXCL16 sense | TGT | GGA | ACT | GGT | CAT | GGG | AAG | | | |
| mCXCL16 anti-sense | TGC | TCG | TGT | CCG | AAG | GTG | T | | | |
| mCXCL16 TaqMan probe | TGC | CTC | AAG | CCA | GTA | CCC | AGA | CCC | | |
| fractalkine sense | CAC | CTC | GGC | ATG | ACG | AAA | T | | | |
| fractalkine anti-sense | TTG | TCC | ACC | CGC | TTC | TCA | A | | | |
| fractalkine TaqMan probe | TGC | TCA | TCC | GCT | ATC | AGC | TAA | ACC | AGG | A |

### (Immunofluorescence histological staining of fractalkine)

Frozen sections with a thickness of 6 µm were prepared from intestinal tracts on day 10 after GVHD induction. Immunofluorescent double staining was performed by using a goat anti-fractalkine antibody (final concentration 5 µg/ml) and a biotin-labeled anti Ly5.1 antibody (final concentration 5 µg/ml) as primary antibodies, and Alexa 546-labeled anti-goat IgG antibody (final concentration 0.5 µg/ml) and Alexa 488-labeled Streptabidin (final concentration 0.5 µg/ml) as secondary antibodies. Further, goat IgG (Chemicon international) was used as a negative control for anti-fractalkine antibody. Expression of fractalkine and its spacial relation with donor cells infiltrated to intestinal tract were analyzed with a confocal laser scanning microscope (Olympus).

### (Anti-fractalkine antibody experiment)

200 µg of anti-fractalkine antibody or hamster IgG (Jackson Immuno Research) were dissolved in 100 µl PBS, and administered intraperitoneally on day 6, 8, 10 after GVHD induction. On day 14, the number of donor CD8 T cells infiltrated to small intestine and liver was measured and the effect of the administration of the anti-fractalkine antibody on organ infiltration by donor CD8 T cells was evaluated. Further, the number of apoptotic cells in small intestine was measured by Tunel staining and the effect of the anti-fractalkine antibody treatment on intestinal injury was evaluated.

### [Result and discussion]

### (Fig. 1)

Correlation between intestinal infiltration by donor CD8 T cells and intestinal injury is shown in Fig. 1. A shows the infiltration by donor CD8 T cells in spleen, mesenteric lymph node, small intestine; B shows the image of intestinal tissue (HE staining) of normal BDF1 and on day 8 and day 12 after induction of GVHD; C shows the detection of apoptotic cells in intestinal tract of normal BDF1 and on day 8, day 12 after induction of GVHD.

From FCM analysis, it was shown that the peak of the donor CD8 T cell infiltration in spleen, mesenteric lymph node is on day 10, while infiltration by donor CD8 T cells in small intestine showed a different bkinetics, starting from day 10, and increasing rapidly toward day 14, which is the peak of intestinal GVHD (Fig. 1-A). Moreover, from HE staining and Tunel staining, particular symptoms in GVHD including hyperplasia of intestinal crypts, increase of apoptotic cells and distruction of crypts were accompanied by infiltration by donor CD8 T cells (Fig. 1-B, C).

From these results, it was revealed that donor CD8 T cells acquire infiltration ability to intestinal tract at the later phase of GVHD, and rapid intestinal infiltration by donor CD8 T cells correlate significantly with the onset of intestinal GVHD.

### (Fig. 2)

Involvement of fractalkine/CX3CR1 in intestinal GVHD is shown in Fig. 2. A shows gene expression of the chemokine receptor in donor CD8 T cells before transger and those infiltrated in intestinal tract and liver on day 10 after GVHD induction. B shows gene expression of the chemokine in intestinal tract and liver of normal and on day 10 after GVHD induction. C shows fractalkine expression and infiltration of donor lymphocytes in intestinal tract on day 10 after GVHD induction.

By investigating molecules associated with intestinal infiltration by donor CD8 T cells in late phase of GVHD by Real-time PCR, it was revealed that expression of the chemokine receptor CX3CR1 in donor CD8 T cells infiltrated in intestinal tract on day 10 after transfer increases significantly and that fractalkine which is a ligand of CX3CR1 is expressed in intestinal tract tissue (Fig. 2-A, B). Specific staining was observed in intestinal epithelial cells and vascular endothelial cells by immunofluorescent histological staining using anti-fractalkine antibody. Moreover, it was observed that Ly5.1-positive donor lymphocytes are adhered to the basal side of the fractalkine positive intestinal epithelial cells (Fig. 2-C).

From these results, it was suggested that in the late phase of GVHD, as donor CD8 T cells increase CX3CR1 expression and acquire an infiltration ability to intestinal tract expressing fractalkine, intestinal infiltration by donor CD8 T cells and intestinal GVHD are induced.

### (Fig. 3)

Suppression of the intestinal infiltration by donor CD8 T cells by administration of anti-fractalkine antibody is shown in Fig. 3.

By administrating anti-fractalkine antibodies on day 6, 8, 10 in the late phase of GVHD, the number of donor CD8 T cells infiltrated to intestinal tract decreased significantly compared to that of the group administered with control antibody. On the other hand, there was no significant difference for donor CD8 T cells infiltrated to liver.

From these results, it was revealed that the fractalkine/CX3CR1 interaction plays a critical role to intestinal infiltration by donor CD8 T cells.

### (Fig. 4)

Improvement of intestinal GVHD by administration of anti-fractalkine antibody is shown in Fig. 4. A shows the detection of apoptotic cells in intestinal crypt by Tunel staining, B shows the number of apoptotic cells per 20 intestinal crypts.

By estimating the effect of the anti-fractalkine antibody administration on intestinal tract tissue injury with the measurement of the number of apoptotic cells by Tunel staining, apoptotic cells in intestinal crypts were decreased in the group administered with anti-fractalkine antibody.

From these results, it was revealed that intestinal tract injury in GVHD was ameliorated by anti-fractalkine antibody administration.

### (Figs. 1-4)

It was revealed from the results of Figs. 1 to 4 that intestinal tract injury in acute GVHD is induced by donor CD8 T cells infiltrating to intestinal tract in the late phase of GVHD, and that the fractalkine/CX3CR1 interaction is involved in intestinal infiltration by donor CD8 T cells, and that intestinal injury in GVHD could be reduced by inhibiting the interaction. Compared with a conventional method using immune suppressor, a method for preventing/treating by targeting fractalkine is a method for suppressing selectively intestinal infiltration by donor CD8 T cells. Moreover, the most severe life-threatening intestinal injuries in GVHD could be treated, even in the later phase of GVHD.

### Industrial Applicability

As well as bone marrow transplantation is a representative treatment method for various hematologic diseases such as chronic myelogenous leukemia, cell transplantation is a useful treatment method in the medical field. However, graft-versus host disease (GVHD) can be a critical obstacle when performing these treatments by transplantation. Currently, non-specifc immunosuppressive agents targeting the proliferating process of immune cells or immunosuppressive agents acting widely are used as standard GVHD preventive protocols. However, as these agents have a non-specific and a wide immunosuppressive effect, their toxicity is high and infectious diseases due to decrease of immunity or recurrence of tumor is becoming a problem. The preventive or therapeutic agent of GVHD, or a method for preventing or treating GVHD of the present invention inhibit specifically the mechanism that develop organ injuries in GVHD, to prevent organ injuries or to exert a therapeutic effect, it becomes possible to avoid GVHD more selectively. In particular, the therapeutic agent of GVHD and the method for treating GVHD of the present invention takes advantage of fractalkine as a target, and effect thereof can be expected even after the onset of GVHD and have a characteristic that is not observed in conventional immunosuppressive agents. Moreover, in the present invention, by using the pathogenic mechanism of organ injuries in GVHD that has been found in the present invention, it is possible to provide a method for screening a substance having specific preventive or therapeutic effect on organ injuries, and to develop preventive and therapeutic agents that avoid GVHD more selectively.

## Claims

1. A method for avoiding preventively or therapeutically an onset or a development of graft-versus-host disease by suppressing specifically an organ infiltration by CD8 T cells.

2. The method for avoiding preventively or therapeutically an onset or a development of graft-versus-host disease according to claim 1, wherein the specific suppression of an organ infiltration by CD8 T cells is inhibition of the interaction between fractalkine and a chemokine receptor CX3CR1 which is expressed on donor CD8 T cells.

3. The method for avoiding preventively or therapeutically an onset or a development of graft-versus-host disease according to claim 2, wherein the inhibition of the interaction between fractalkine and the chemokine receptor CX3CR1 is inhibition of a function of fractalkine and/or a chemokine receptor CX3CR1 which is expressed on donor CD8 T cells.

4. The method for avoiding preventively or therapeutically an onset or a development of graft-versus-host disease according to claim 2 or 3, wherein the inhibition of the interaction between fractaline and the chemokine receptor CX3CR1 which is expressed on donor CD8 T cells is the inhibition of the interaction using an anti-fractalkine antibody.

5. The method for avoiding preventively or therapeutically an onset or a development of graft-versus-host disease according to claim 4, wherein the anti-fractalkine antibody is a monoclonal antibody.

6. The method for avoiding preventively or therapeutically an onset or a development of graft-versus-host disease according to any one of claims 1 to 5, wherein the specific suppression of an organ infiltration by CD8 T cells is an infiltration by cytotoxic CD8 T cells to intestinal tract.

7. A preventive or therapeutic agent specific to an organ injury in graft-versus-host disease, comprising a substance inhibiting an interaction between fractalkine and the chemokine receptor CX3CR1 which is expressed on donor CD8 T cells as an active component.

8. The preventive or therapeutic agent specific to an organ injury in graft-versus-host disease according to claim 7, wherein the substance inhibiting an interaction between fractalkine and the chemokine receptor CX3CR1 which is expressed on donor CD8 T cells is a substance inhibiting a function of fractalkine and/or the chemokine receptor CX3CR1 which is expressed on donor CD8 T cells.

9. The preventive or therapeutic agent specific to an organ injury in graft-versus-host disease according to claim 7 or 8, wherein the substance inhibiting the interaction between fractaline and the chemokine receptor CX3CR1 which is expressed on donor CD8 T cells is an anti-fractalkine antibody.

10. The preventive or therapeutic agent specific to an organ injury in graft-versus-host disease according to claim 9, wherein the anti-fractalkine antibody is a monoclonal antibody.

11. A method for screening a substance having a preventive or therapeutic effect specific to an organ injury in graft-versus-host disease, comprising the steps of administering a test substance to a model animal with graft-versus-host disease transferred with donor CD8 T cells, and detecting/evaluating an infiltration by donor CD8 T cells to organs.

12. The method for screening a substance having a preventive or therapeutic effect specific to an organ injury in graft-versus-host disease according to claim 11, wherein the infiltration by donor CD8 T cells to organs is determined by detecting/evaluating the expression of the chemokine receptor CX3CR1 and/or fractalkine in donor CD8 T cells.

13. The method for screening a substance having a preventive or therapeutic effect specific to an organ injury in graft-versus-host disease according to claim 11 or 12, wherein the infiltration by donor CD8 T cells to organs is an infiltration by donor CD 8 T cells to intestinal tract.

14. The method for screening a substance having a preventive or therapeutic effect specific to an organ injury in graft-versus-host disease according to any one of claims 11 to 13, wherein the model animal with graft-versus-host disease transferred with donor CD8 T cells is a model mouse with graft-versus-host disease transferred with donor CD8 T cells.

15. A preventive or therapeutic agent specific to an organ injury in graft-versus host disease comprising a substance obtained by the method for screening substances having a preventive or therapeutic effect specific to an organ injury in graft-versus-host disease according to any one of claims 11 to 14 as an active component.
